# EUROPEAN PATENT SPECIFICATION

(11) **EP 1 773 133 B1**
(45) Date of publication and mention of the grant of the patent: **29.10.2008**
(21) Application number: 05746762.3
(22) Date of filing: 05.05.2005
(51) Int. Cl.: A23K 1/16

(54) **PROCESSES FOR PREPARING PLANT MATTER EXTRACTS AND PET FOOD COMPOSITIONS**
VERFAHREN ZUR HERSTELLUNG VON PFLANZENEXTRAKTEN UND HEIMTIERFUTTERMITTELN
PROCEDES POUR PREPARER DES EXTRAITS DE MATIERES VEGETALES ET COMPOSITIONS ALIMENTAIRES POUR ANIMAUX DOMESTIQUES

(30) Priority: 10.05.2004 US 842300
(43) Date of publication of application: 18.04.2007
(73) Proprietor: THE IAMS COMPANY, Dayton, Ohio 45414 (US)
(72) Inventor: MASSIMINO, Stefan Patrick, Kettring, Ohio 45429 (US); NIEHOFF, Raymond Louis, Hamilton, OH 45013 (US); SARAMA, Robert Joseph, Loveland, OH 45140 (US); TRIBELHORN, Ronald Eugene, Clayton, OH 45315 (US)
(74) Representative: Goodier, Claire-Louise
(86) International application number: PCT/US2005/016038
(87) International publication number: WO 2005/110036

(56) References cited:
- WO-A-20/04100670
- US-A1- 2002 035 071
- US-A1- 2003 092 669
- LIU XUAN ET AL: "Postulated physiological roles of the seven-carbon sugars, mannoheptulose, and perseitol in avocado" JOURNAL OF THE AMERICAN SOCIETY FOR HORTICULTURAL SCIENCE, vol. 127, no. 1, January 2002 (2002-01), pages 108-114, XP002358592 ISSN: 0003-1062
- SHAW P E ET AL: "HIGH PERFORMANCE LIQUID CHROMATOGRAPHIC ANALYSIS OF D MANNO HEPTULOSE PERSEITOL GLUCOSE AND FRUCTOSE IN AVOCADO CULTIVARS" JOURNAL OF AGRICULTURAL AND FOOD CHEMISTRY, AMERICAN CHEMICAL SOCIETY. WASHINGTON, US, vol. 28, no. 2, 1980, pages 379-382, XP009053733 ISSN: 0021-8561

## Description

### FIELD OF THE INVENTION

The present invention is directed to processes for preparing a plant extract, including processes for preparing pet food compositions containing such extracts. In certain embodiments, the extracts or pet food compositions prepared thereby may comprise a selected carbohydrate component, or a plant material selected from avocado, alfalfa, fig, primrose, and mixtures thereof.

### BACKGROUND OF THE INVENTION

Studies have indicated that restriction of caloric intake by food deprivation slows down certain undesirable cellular processes in laboratory animals, many associated with aging and age-related diseases.

In particular, caloric restriction has been shown to consistently extend the life span, delay onset and slow tumor progression, and retard physiologic aging in many systems. Indeed, research spanning more than sixty years has shown that caloric restriction is a nutritional intervention that consistently extends longevity in animals. See Weindruch and Walford, "The Retardation of Aging and Disease by Dietary Restriction," Springfield, IL: Charles C. Thomas (1988); Yu, "Modulation of Aging Processes by Dietary Restriction," Boca Raton: CRC Press (1994); and Fishbein, "Biological Effects of Dietary Restriction," Springer, New York (1991). These effects of caloric restriction on life span and tumorigenesis have been reported numerous times since the early studies of McKay. See McKay et al., "The Effect of Retarded Growth Upon the Length of Lifespan and Upon Ultimate Body Size," J. Nutr., Vol. 10, pp. 63 - 79 (1935). Indeed, over the past two decades, a resurgence of interest in caloric restriction in gerontology has led to the general acceptance that this dietary manipulation slows physiologic aging in many systems. See Weindruch and Walford, "The Retardation of Aging and Disease by Dietary Restriction," Springfield, IL: Charles C. Thomas (1988); Yu, "Modulation of Aging Processes by Dietary Restriction," Boca Raton: CRC Press (1994); and Fishbein, "Biological Effects of Dietary Restriction," Springer, New York (1991).

Reductions in fasting glucose and insulin levels are readily measured biomarkers of caloric restriction. Calorically restricted rodents exhibit lower fasting glucose and insulin levels, and the peak glucose and insulin levels reached during a glucose challenge are reduced in those on caloric restriction. See Kalant et al., "Effect of Diet Restriction on Glucose Metabolism and Insulin Repsonsiveness and Aging Rats," Mech. Aging Dev., VoL 46, pp. 89 - 104 (1988). It is also known that hyperinsulinemia is a risk factor associated with several such disease processes, including heart disease and diabetes (Balkau and Eschwege, Diabetes Obes. Metab. 1 (Suppl. 1): S23 - 31, 1999). Reduced insulin levels and body temperature are two of the most reliable indicators of this altered metabolic profile (Masoro et al., J. Gerontol. Biol. Sci. 47;B202-B208, 1992); Koizumi et al., J. Nutr. 117: 361 - 367, 1987; Lane et al., Proc. Nat. Acad. Sci. 93:4154 - 4164, 1996).

Components such as 2-deoxy-D-glucose have been described which block or inhibit certain aspects of carbohydrate metabolism and may therefore mimic the effects of caloric restriction (Rezek et al., J. Nutr. 106:143 - 157, 1972; U.S. Patent Application Publication No. 2002/0035071). These components exert a number of physiological effects, including reduction of body weight, decrease in plasma insulin levels, reduction of body temperature, retardation of tumor formation and growth, and elevation of circulating glucocorticoid hormone concentrations. (For a review see Roth et al., Ann. NY Acad. Sci. 928:305 - 315, 2001). These effects result from inhibition of carbohydrate metabolism.

Liu et al (J. Amer. Soc. Hort. Sci. 127(1): 108-114, 2002) disclosed how to extract and quantify the production of seven-carbon ketosugar, such mannoheptulose, in mature leaves of avocado trees.

Extraction and high-performance liquid chromatography of various cultivars of avocado growing in Florida has clarified that the levels of monosaccharides and parseitol are correlated with varieties, production area and ripering procedures.

US 2003/0092669 a dietary supplement comprising mannoheptulose, which is capable of decreasing serum insulin levels in humans.

WO 2004/100670 discloses a method of processing avocado meat comprising heating the avocado to denature native degradative enzymes while the avocado meat is in an environment having pH greater than 8.

However, the commercial utility of such components has been limited, particularly since commercial processes of preparing such components had yet been identified. The present inventors herein provide processes for preparation of compositions comprising a plant matter extract containing such a component. In particular, it has been found that the components herein may be processed from plant matter, and then optionally subjected to traditional food processing (such as, for example, extrusion or other such vigorous methods) without compromising the integrity of the component.

### SUMMARY OF THE INVENTION

The present invention is directed to processes for preparing a a food composition.

The processes for preparing a food composition comprise:
(a) providing plant matter selected from the group consisting of avocado, alfalfa, fig, primrose, and mixtures thereof;
(b) combining the plant matter with an aqueous solution and optionally with an enzyme, further optionally with heating, to provide a digested plant mixture;
(c) separating fractions present in the digested plant mixture by filtration to provide a carbohydrate extract as the resulting filtrate;
(d) concentrating the carbohydrate extract to enhance the concentration of carbohydrate therein; and
(e) combining the carbohydrate extract with one or more food composition components.

### DETAIL DESCRIPTION OF THE INVENTION

Various documents including, for example, publications and patents, are recited throughout this disclosure. All such documents are hereby incorporated by reference. The citation of any given document is not to be construed as an admission that it is prior art with respect to the present invention.

All percentages and ratios are calculated by weight unless otherwise indicated. All percentages and ratios are calculated based on the total composition unless otherwise indicated.

Referenced herein are trade names for components including various ingredients utilized in the present invention. The inventors herein do not intend to be limited by materials under a certain trade name. Equivalent materials (e.g., those obtained from a different source under a different name or reference number) to those referenced by trade name may be substituted and utilized in the descriptions herein.

In the description of the invention various embodiments or individual features are disclosed. As will be apparent to the ordinarily skilled practitioner, all combinations of such embodiments and features are possible and can result in preferred executions of the present invention.

The compositions herein may comprise, consist essentially of, or consist of any of the features or embodiments as described herein.

As will also be apparent, all combinations of the embodiments and features taught in the foregoing disclosure are possible and can result in preferred executions of the invention.

As used herein, the term "pet" means a domestic dog or cat.

### Processes of the Present Invention

The present invention is directed to processes for preparing a food composition by providing plant matter. In one embodiment, the food composition is a pet food composition.

The processes for preparing a food composition comprise:
(a) providing plant matter selected from the group consisting of avocado, alfalfa, fig, primrose, and mixtures thereof;
(b) combining the plant matter with an aqueous solution and optionally with an enzyme, further optionally with heating, to provide a digested plant mixture;
(c): separating fractions in the digested plant mixture by filtration to provide a carbohydrate extract as the resulting filtrate;
(c) concentrating the carbohydrate extract to enhance the concentration of carbohydrate therein; and
(d) combining the carbohydrate extract with one or more food composition components.

The plant matter provided in the various processes may be avocado, alfalfa, fig, primrose, and mixtures thereof. These plants are known to contain carbohydrate components such as 2-deoxy-D-glucose, 5-thio-D-glurose, 3-O-methylglucose, 1,5-anhydro-D-glucitol, 2,5-anhydro-D-mannitol, or mannoheptulose. See e.g., U.S. Patent Application Publication No. 2002/0035071. Processes resulting in extracts which have elevated concentrations of mannoheptulose are preferred herein. Advantageously, the mannoheptulose or any other component may be present as a component of plant matter such as avocado, or other enriched source of mannoheptulose such as alfalfa, fig, or primrose.

The plant matter may be any portion or whole of the plant, such as the leaves, fruit, seed or pit, particularly at least those portions of the plant that contain elevated levels of carbohydrate component. The inclusion of one or more of these components as part of the extracts of food compositions herein are useful, for example, to alter utilization of glucose or other energy sources and to mimic metabolic effects of caloric restriction. Without intending to be limited by theory, the present use of glucose anti-metabolites to alter glucose metabolism serves to lower the metabolic rate through inhibition of glucose as an energy source on the cellular level. Judicious use of components that block the normal metabolism of cellular glucose can result in changes in physiological function that are similar to those arising from caloric restriction. Caloric restriction has been consistently shown to extend longevity in animals. See Weindruch and Walford, "The Retardation of Aging and Disease by Dietary Restriction," Springfield, IL: Charles C. Thomas (1988); Yu, "Modulation of Aging Processes by Dietary Restriction," Boca Raton: CRC Press (1994); and Fishbein, "Biological Effects of Dietary Restriction," Springer, New York (1991).

The plant matter may include the fruit, seed (or pit), branches, leaves, or any other portion of the relevant plant or combination thereof. For example, in one embodiment herein, the avocado is provided, and the process may commence with whole or partial avocado fruit, including the pit or devoid (or partially devoid) of the pit. If the plant matter which is provided contains a pit, or partial pit, the pit or portion thereof may be optionally removed prior to further processing. Alfalfa, fig, or primrose may be similarly processed.

Avocado (also commonly referred to as alligator pear, aguacate, or palta) contains unusually enriched sources of mannoheptulose, as well as related sugars and other carbohydrate components. Avocado is a sub-tropical evergreen tree fruit, growing most successfully in areas of California, Florida, Hawaii, Guatemala, Mexico, the West Indies, South Africa, and Asia.

Species of avocado include, for example, *Persea Americana* and *Persea nubigena,* including all cultivars within these illustrative species. Cultivars may include 'Anaheim,' 'Bacon,' 'Creamhart,' 'Duke,' 'Fuerte,' 'Ganter,' 'Gwen,' 'Hass,' 'Jim,' 'Lula,' 'Lyon,' 'Mexicola,' 'Mexicola Grande,' 'Murrieta Green,' 'Nabal,' 'Pinkerton,' 'Queen,' 'Puebla,' 'Reed,' 'Rincon,' 'Ryan,' 'Spinks,' 'Topa Topa,' 'Whitsell,' 'Wurtz,' and 'Zutano.' The fruit of the avocado is particularly preferred for use herein, which may contain the pit or wherein the pit is removed or at least partially removed. Fruit from *Persea Americana* is particularly preferred for use herein, as well as fruit from cultivars which produce larger fruits (*e.g*., about 12 ounces or more when the fruit is mature), such as Anaheim, Creamhart, Fuerte, Hass, Lula, Lyon, Murrieta Green, Nabal, Queen, Puebla, Reed, Ryan, and Spinks.

Plant matter from alfalfa, fig, or primrose are also reported to provide relatively high levels of mannoheptulose. Alfalfa is also referred to as *Medicago sativa.* Fig, or *Ficus carica* (including Cluster fig or Sycamore fig, for example) may also be used, as well as primrose or *Primula officinalis.*

In one embodiment, production of a digested plant mixture comprises combination of the plant matter with an aqueous solution, such as water, to assist with maceration of the plant into manageable constituents. Optionally but preferably, an enzyme having cellulose or pectin activity, or any combination thereof (such as a cellulase, hemicellulase, or pectinase) is included to assist with such maceration, including to assist with dissolution and release of carbohydrates via cell wall disruption. The utility of such an enzymatic treatment may be enhanced through heating during such maceration, such as from above ambient temperature to 120 °C, or to 100 °C, or from 60 °C to 120 °C, or from 60 °C to 100 °C. Agitation is further preferably utilized, typically for up to 24 hours, but dependent upon the batch under processing. In one embodiment, the pH is controlled such to preserve enzyme activity, often in the range of pH from 4 to 6, preferably in the range of pH from 5 to 6. As such, depending upon such factors as ripeness of plant matter, quality of process aqueous solution (such as water added for process, for example), amounts of acid or base may be desirable as will be appreciated by one of ordinary skill in the art.

Optionally, to assist with deactivation of the enzymes present, heating may be increased at the time of, or after, initial heating and agitation to form the digested plant mixture. Water is optionally heated to processing temperatures prior to the addition of the plant matter. Heat may be applied by a jacketed tank where low pressure steam is utilized.

The digested plant mixture results in fractions which are separated in accordance with common techniques. The fractions present in the digested plant mixture are separated by filtration to provide the carbohydrate extract as the resulting filtrate, with the filter cake being discarded. Other methods may include, but not be limited to, gravimetric, centrifugal, other filtrations, or combinations thereof.

The carbohydrate extract is then concentrated utilizing at least one concentration method selected from the group consisting of heating, vacuum drying, evaporation, refractance window drying, freeze drying, spray drying, any other useful technique, or any combination of the foregoing. In one embodiment, at least one technique such as refractance window drying is used.

Once concentrated, the carbohydrate extract is utilized in the food compositions of the present invention. In one embodiment herein, the present processes result in preferred yields of mannoheptulose or other carbohydrate, or carbohydrate extract, based on the starting mass of the plant matter (*e.g*., avocado). In one embodiment, the yield of mannohaptulose present in the carbohydrate extract subsequent to concentration is less than 20%, or from 0.1% to 10%, or from 1% to 7%, based on the starting mass of the plant matter. In another embodiment, the yield of the carbohydrate extract subsequent to concentration is less than 30%, or from 5% to 25%, or from 8% to 20%, based on the starting mass of the plant matter. Of course, even higher yields may be desirable, and lower yields may also be acceptable.

Food compositions may be prepared in accordance with the present processes by further including combining the digested plant mixture with one or more food composition components. As used herein, the term "food composition" means a composition that is intended for ingestion by an animal, such as a human, or other animal (including a pet). For example, a pet food composition is a food composition that is intended for ingestion by a pet. Food compositions are widely known in the art- Pet food compositions may include, without limitation, nutritionally balanced compositions suitable for daily feed, as well as supplements (*e.g*., treats) which may or may not be nutritionally balanced. As used herein, the term "nutritionally balanced," with reference to the pet food composition, means that the composition has known required nutrients to sustain life in proper amounts and proportion based on recommendations of recognized authorities in the field of pet nutrition.

In one embodiment herein, the inventive processes are utilized to prepare a food composition comprising a component selected from 2-deoxy-D-glucose; 5-thio-D-glucose; 3-O-methylglucose; 1,5-anhydro-D-glucitol; 2,5-anhydro-D-mannitol; mannoheptulose; and mixtures thereof. In a further embodiment, such component is present in the prepared composition at particular levels. In particular, it has been found that relatively low levels, as well as relatively high doses of the component, while useful, may provide less than optimal efficacy for desired purposes. In particular, an optional dose to the pet, on a daily basis, has been surprisingly discovered to be from 1 mg/kg to 15 mg/kg, more advantageously from 2 mg/kg to 10 mg/kg, even more advantageously from 2 mg/kg to 5 mg/kg, wherein (as will be commonly understood in the art) the "mg" refers to level of the component and the "kg" refers to kilograms of the pet. In certain embodiments, this may translate to preparation of pet food compositions comprising less than 5%, or less than 2%, or from 0.0001% to 0.5% of the component, all by weight of the composition. The level of component may be determined by one of ordinary skill in the art based on a variety of factors, for example, the form of the pet food composition (*e.g*., whether a dry composition, semi-moist composition, wet composition, or supplement, or any other form or mixture thereof). The ordinarily skilled artisan will be able to utilize the preferred optimal doses, and use these to determine the optimal level of component within a given pet food composition.

Similarly, optimal levels of plant matter extract will of course be dependent upon level of efficacious component within such extract. Optimal extracts have been found herein which comprise from 1% to 99% of the component, alternatively from 5% to 75% of the component, alternatively from 10% to 50% of the component, all by weight of the extract.

Food compositions will contain one or more food composition components, which will of course be widely known in the art.

For example, pet food compositions may advantageously include food composition components intended to supply necessary dietary requirements, as well as treats (*e.g*., dog biscuits) or other food supplements. Optionally, the composition herein may be a pet food composition such as a dry composition (for example, kibble), semi-moist composition, wet composition, or any mixture thereof Alternatively or additionally, the composition is a supplement, such as a gravy, drinking water, yogurt, powder, suspension, chew, treat (*e.g*., biscuits) or any other delivery form.

In one embodiment, the food compositions may comprise, on a dry matter basis, from 10% to 90% crude protein, alternatively from 20% to 50% crude protein, alternatively from 20% to 40% crude protein, by weight of the food composition, or alternatively from 20% to 35% crude protein. The crude protein material may comprise vegetable proteins such as soybean, cottonseed, and peanut, or animal proteins such as casein, albumin, and meat protein. Non-limiting examples of meat protein useful herein include a protein source selected from the group consisting of beef, pork, lamb, poultry, fish, vegetable, and mixtures thereof.

Furthermore, the compositions may comprise, on a dry matter basis, from 5% to 40% fat, alternatively from 10% to 35% fat, by weight of the food composition.

The compositions of the present invention may further comprise a source of carbohydrate. Grains or cereals such as rice, corn, milo, sorghum, barley, wheat, and the like are illustrative sources.

The compositions may also contain one or more other materials such as dried whey and other dairy by products.

### EXAMPLES

The following examples are provided to illustrate the invention and are not intended to limit the scope thereof in any manner.

### Example 1

Avocado extract containing enhanced levels of mannoheptulose is prepared in accordance with the following process, and utilized in pet food compositions:

Whole avocado fruit (900 kilograms) is provided. The fruit is split and the pits are removed, either partially or wholly, providing 225 kilograms of pitted avocado halves. The raw avocado is charged to a disintegrator, whereupon some agitation, water (3000 kilograms) and CELLUBRIX (commercially available from Novozymes A/S) (1 liter) is further charged. The mixture is further agitated and concurrently heated to 66°C. Upon completion of the charge, further CELLUBRIX (about 1 liter) is added, and the entire mixture is held under agitation for 12 hours at a controlled pH of 5.5. The temperature is then further increased to 80°C and then held for at least 2 hours. The resulting digested plant mixture is then filtered at 80°C to provide the carbohydrate extract as the filtrate. The carbohydrate extract is then evaporated in a simplified recirculation system at 80 °C, under vacuum, to a provide the carbohydrate extract having about from 10% to 20% solids and a pH of 5.5. The extract is then further concentrated using a refractance window dryer to provide 100 kilograms of the extract as a crystalline or powder (a yield of 11% carbohydrate extract, based on the starting mass of the whole avocado fruit, which is analyzed as a yield of 4.5% mannoheptulose, based on the starting mass of the whole avocado fruit). The carbohydrate extract may be used in the pet food compositions of the present invention.

### Example 2

Two kibble compositions having the following components at the approximate indicated amounts are prepared using methods which are standard in the art, including extrusion, and are fed to cats as a daily feed:

| Component | Example 1A (Component Amount indicated as Wt%) | Example 1B (Component Amount indicated as Wt%) |
|---|---|---|
| Extract of Avocado Prepared in Accordance with Present Invention | 0.02 | 0.01 |
| Chicken, Chicken By-product Meal, and Fish Meal | 44 | 47 |
| Chicken Fat | 8 | 6 |
| Beet Pulp | 2 | 3 |
| Salts | 2.5 | 2 |
| Vitamins and Minerals** | 1 | 1 |
| Minors | 3.5 | 4 |
| Grains (corn, sorghum) | Remainder | Remainder |

| | | |
|---|---|---|
| * Avocado may be substituted with other plant matter having enhanced mannoheptulose content. **Vitamin and Minerals include: Vitamin E, beta-caurotene and Vitamin A, Zinc Oxide, Ascorbic Acid, Manganese Sulfate, Copper Sulfate, Manganous Oxide, Calcium Pantothenate, Biotin, Vitamin B₁₂, Vitamin B₁, Niacin, Vitamin B₂, Vitamin B₆, Vitamin D₃, Folic Acid. | | |

### Example 3

A beef-flavor gravy composition is prepared by combining the following components in a conventional manner:

| **Component** | **Wt%** |
|---|---|
| Extract of Avocado Prepared in Accordance with Present Invention | 0.14 |
| Chicken Fat | 3.0 |
| Spray-Dried Beef Particles and Broth | 3.0 |
| Xanthan Gum | 0.5 |
| Flax Seed | 0.2 |
| Vegetables | 0.2 |
| Vitamins** | 0.06 |
| Minerals | 0.04 |
| Phosphoric Acid | 0.95 |
| Beef Flavor | 0.1 |
| Water | Remainder |

| | |
|---|---|
| **Vitamins and Minerals include: Vitamin E, beta-carotene and Vitamin A, Zinc Oxide, Ascorbic Acid, Manganese Sulfate, Copper Sulfate, Manganous Oxide, Calcium Pantothenate, Biotin, Vitamin B₁₂, Vitamin B₁, Niacin, Vitamin B₂, Vitamin B₆, Vitamin D₃, Folic Acid. | |

One fluid ounce of the gravy composition is admixed with one-half cup of standard dog kibble diet daily prior to feeding to a dog. Amounts of the gravy composition are determined as desired by the guardian of the dog.

## Claims

1. A process for preparing a food composition comprising:
(a) providing plant matter selected from the group consisting of avocado, alfalfa, fig, primrose, and mixtures thereof;
(b) combining the plant matter with an aqueous solution and optionally with an enzyme, and further optionally with heating, to provide a digested plant mixture;
(c) separating fractions present in the digested plant mixture by filtration to provide the carbohydrate extract as the resulting filtrate;
(d) concentrating the digested plant mixture to enhance the concentration of carbohydrate therein; and
(e) combining the carbohydrate extract with one or more food composition components.

2. The process according to Claim 1 wherein the plant matter comprises fruit of avocado and is combined with the aqueous solution and the enzyme, with heating, to provide the digested plant mixture.

3. The process according to any of the preceding claims wherein the heating is at a temperature which is from above ambient temperature to 120 °C.

4. The process according to any of the preceding claims wherein subsequent to providing the plant matter, wherein the fruit of avocado comprises a pit, the process further comprises at least partially removing the pit.

5. The process according to any of the preceding claims wherein the enzyme is a cellulase enzyme.

6. The process according to any of the preceding claims wherein the carbohydrate extract is concentrated utilizing at least one concentration method selected from the group consisting of heating, vacuum drying, refractance window drying, freeze drying, and spray drying.

7. The process according any of the preceding claims wherein the yield of mannoheptulose present in the plant matter extract is less than 20%, based on the starting mass of the plant matter.

8. The process according to claim 1, wherein the food composition components comprise pet food composition components.

## Patentansprüche

1. Verfahren zur Herstellung einer Nahrungszusammensetzung, umfassend:
(a) Bereitstellen von Pflanzenmaterial, ausgewählt aus der Gruppe bestehend aus Avocado, Alfalfa, Feige, Primel und deren Mischungen;
(b) Kombinieren des Pflanzenmaterials mit einer wässrigen Lösung und optional mit einem Enzym, ferner optional unter Erwärmen, um eine digerierte Pflanzenmischung bereitzustellen;
(c) Trennen von Fraktionen, die in der digerierten Pflanzenmischung vorhanden sind, durch Filtration, um den Kohlenhydratextrakt als das resultierende Filtrat bereitzustellen;
(d) Aufkonzentrieren der digerierten Pflanzenmischung, um die Konzentration des Kohlenhydrats darin zu verstärken;
(e) Neukombinieren des Kohlenhydratextrakts mit einem oder mehreren Nahrungszusammensetzungs-Bestandteilen.

2. Verfahren nach Anspruch 1, wobei das Pflanzenmaterial Avocadofrucht umfasst und mit der wässrigen Lösung und dem Enzym, unter Erwärmen, kombiniert wird, um die digerierte Pflanzenmischung bereitzustellen.

3. Verfahren nach einem der vorstehenden Ansprüche, wobei das Erwärmen bei einer Temperatur stattfindet, die von etwa Umgebungstemperatur bis etwa 120 °C reicht.

4. Verfahren nach einem der vorstehenden Ansprüche, wobei anschließend an die Bereitstellung des Pflanzenmaterials, wobei die Avocadofrucht einen Kern aufweist, das Verfahren ferner das zumindest teilweise Entfernen des Kerns umfasst.

5. Verfahren nach einem der vorstehenden Ansprüche, wobei das Enzym ein Cellulaseenzym ist.

6. Verfahren nach einem der vorstehenden Ansprüche, wobei der Kohlenhydratextrakt unter Verwendung mindestens eines Aufkonzentrierungsverfahrens, das ausgewählt ist aus der Gruppe bestehend aus Erwärmen, Vakuumtrocknung, Refractance-Window-Trocknung, Gefriertrocknung und Sprühtrocknung, aufkonzentriert wird.

7. Verfahren nach einem der vorstehenden Ansprüche, wobei die Ausbeute an Mannoheptulose, die in dem Pflanzenmaterial vorhanden ist, weniger als 20 % ist, bezogen auf die Ausgangsmasse des Pflanzenmaterials.

8. Verfahren nach Anspruch 1, wobei die Nahrungszusammensetzungsbestandteile Tierfutterzusammensetzungsbestandteile umfassen.

## Revendications

1. Procédé pour préparer une composition alimentaire comprenant :
(a) la fourniture d'une matière végétale choisie dans le groupe constitué d'avocat, luzerne, figue, primevère, et leurs mélanges ;
(b) la combinaison de la matière végétale avec une solution aqueuse et facultativement avec une enzyme, et encore facultativement avec chauffage, pour fournir un mélange végétal digéré ; et
(c) la séparation des fractions présentes dans le mélange végétal digéré par filtration pour fournir l'extrait d'hydrate de carbone en tant que le filtrat résultant ;
(d) la concentration du mélange végétal digéré pour renforcer la concentration d'hydrate de carbone dedans.
(e) la combinaison de l'extrait d'hydrate de carbone avec un ou plusieurs composants de composition alimentaire.

2. Procédé selon la revendication 1, dans lequel la matière végétale comprend un fruit d'avocat et est combinée avec la solution aqueuse et l'enzyme, avec chauffage, pour fournir le mélange végétal digéré.

3. Procédé selon l'une quelconque des revendications précédentes, dans lequel le chauffage est à une température qui va de plus de la température ambiante à environ 120 °C.

4. Procédé selon l'une quelconque des revendications précédentes, dans lequel après avoir fourni la matière végétale, dans laquelle le fruit d'avocat comprend un noyau, le procédé comprend en outre une élimination au moins partielle du noyau.

5. Procédé selon l'une quelconque des revendications précédentes, dans lequel l'enzyme est une enzyme cellulase.

6. Procédé selon l'une quelconque des revendications précédentes, dans lequel l'extrait d'hydrate de carbone est concentré en utilisant au moins un procédé de concentration choisi dans le groupe constitué de chauffage, séchage sous vide, séchage par fenêtre de réfraction, séchage par gel et séchage par atomisation.

7. Procédé selon l'une quelconque des revendications précédentes, dans lequel la production de mannoheptulose présente dans l'extrait de matière végétale est moins de 20 %, basée sur la masse de départ de la matière végétale.

8. Procédé selon la revendication 1, dans lequel les composants de composition alimentaire comprennent des composants de composition alimentaire pour animaux de compagnie.
